# EUROPEAN PATENT APPLICATION

(11) **EP 1 839 698 A1**
(43) Date of publication of application: **03.10.2007**
(21) Application number: 05806212.6
(22) Date of filing: 10.11.2005
(51) Int. Cl.: A61M 39/02

(54) **TUBE CONNECTION APPARATUS**

(30) Priority: 11.11.2004 JP 2004327481; 22.03.2005 JP 2005081072
(71) Applicant: Nemoto Kyorindo Co., Ltd., Tokyo 113-0033 (JP)
(72) Inventor: NEMOTO, Toru, Nemoto Kyorindo Co., Ltd., Tokyo, 113-0033 (JP); IKOMA, Tomoyuki, Nemoto Kyorindo Co., Ltd., Tokyo, 113-0033 (JP); FUKUDA, Takashi, Nemoto Kyorindo Co., Ltd., Tokyo, 113-0033 (JP)
(74) Representative: Burbaud, Eric
(86) International application number: PCT/JP2005/020606
(87) International publication number: WO 2006/051855

(57) **Abstract**

A tube connection apparatus enabling the connection of tube members to a chemical solution container for a radioactive contrast medium with exposure of a operator to the radiation minimized. When the container (400) is loaded to a container holding mechanism (315) located at a retreat position, the container (400) is moved to a needle insertion position by a container moving mechanism (310) and short/long needle members (341, 342) are moved to the insertion position by a holder moving mechanism (320). Since the short/long needle members (341, 342) are inserted into the elastic member (402) of the chemical liquid container (400) without requiring manual operation by a operator, a liquid supply tube (120) and a chemical solution injection tube (130) can be connected to the container (400) with the exposure of the operator to radiation minimized.

## Description

### Technical Field

The present invention relates to a tube connection apparatus for connecting a tube member to a chemical solution container, and more particularly, to a tube connection apparatus for connecting a liquid supply tube and a chemical solution injection tube to a chemical solution container which contains a radioactive contrast medium.

### Background Art

Presently available imaging diagnostic apparatuses for capturing diagnostic images of patients include CT (Computed Tomography) scanners, MRI (Magnetic Resonance Imaging) apparatuses, PET (Positron Emission Tomography) apparatuses, SPECT (Single Photon Emission Computed Tomography) apparatuses, ultrasonic diagnostic apparatuses, angiography apparatuses, MRA (MR angiography) apparatuses and the like.

When such imaging diagnostic apparatuses are used, a chemical solution such as a contrast medium may be injected into a patient. The chemical solution injectors for automatically performing the injection have been put into practical use. Such a chemical solution injector has an injection execution head on which a chemical solution syringe is removably mounted.

The chemical solution syringe includes a cylinder member formed in a cylindrical shape and a piston member. The cylinder member has a distal end having a hole formed therein, and a proximal end opened. The piston member is slidably inserted into the cylinder member through the opening. Typically, the hole at the distal end is formed in a protruding conduit portion, and a proximal end of an extension tube is connected to it.

The extension tube has a distal end, on which an injection needle is connected, and the injection needle is connected to a blood vessel of a patient. When the chemical solution injector is used, the cylinder member of the chemical solution syringe filled with a chemical solution, is connected to the patient via the extension tube as described above, and the chemical solution syringe is put on the injection execution head of the chemical solution injector.

In the typical chemical solution injector, the injection execution head has a concave portion formed in its upper surface, fitting the cylinder member. The cylinder member is put in the concave portion, to hold the chemical solution syringe. The chemical solution injector holds the piston member by a piston driving mechanism independently of the cylinder member. The piston driving mechanism pushes the piston member, thereby allowing the chemical solution to be injected from the chemical solution syringe into the patient.

There are a pre-filled type and a refill type in the abovementioned chemical solution syringe. The liquid syringe of the pre-filled type includes a cylinder member filled with a chemical solution and is wholly sealed by a packing material for shipment. The chemical solution syringe of the refill type includes a cylinder member, which can be filled with a desired chemical solution by a user. In this case, the chemical solution syringe of the refill type is connected to a chemical solution tank of large volume via an extension tube, and the piston member is pulled from the cylinder member of the chemical solution syringe by a chemical solution injector or the like, to fill the chemical solution into the chemical solution syringe from the chemical solution tank.

When a contrast medium is injected to a patient, the contrast medium is first injected up to a given quantity, and then a physiological saline is injected up to a given quantity, in many cases. The chemical solution injector for performing this operation, is provided to hold a chemical solution syringe filled with the contrast medium, and a chemical solution syringe filled with physiological saline in parallel, in which the chemical solution syringe filled with the contrast medium is first driven, and then the chemical solution syringe filled with the physiological saline is driven. In such a chemical solution injector, since only the appropriate quantity of the contrast medium can be injected into a body part to be imaged, the consumption of the expensive contrast medium can be reduced and the burden on the body of the patient can be alleviated.

In RI (Radio Isotope) tests with the abovementioned PET (Positron Emission Tomography) apparatuses or SPECT (Single Photon Emission Computed Tomography) apparatuses, a radioisotopic chemical solution is used as the contrast medium. Since the contrast medium of this type emits harmful radiation, a syringe cover made of tungsten (or another radiation shielding material such as lead) for blocking radiation is put on the chemical solution syringe, to prevent radiation exposure of an operator. Thus, in the chemical solution injector used in the RI tests, the syringe driving mechanism holds the cylinder member of the chemical solution syringe covered with the syringe cover.

In the RI tests, since the radioisotope in the contrast medium decays over time, the contrast medium needs to be produced, by a dedicated chemical solution producing apparatus, and filled into the chemical solution syringe immediately, before the injection into a patient. In this case, a rubber cap is often attached on the conduit of the syringe, and the sharp injection needle of the chemical solution producing apparatus is inserted into the rubber cap chemical solution. Then, the radioisotopic contrast medium is injected into the syringe from the chemical solution producing apparatus. For injecting the contrast medium from the chemical solution syringe into the patient, an operator detaches the rubber cap from the chemical solution syringe, to connect the extension tube thereto.

Conventionally, the radioactive contrast medium would be filled into the chemical solution syringe by the chemical solution producing apparatus in a medical facility where the contrast medium is injected into a patient. In recent years, however, a system for delivering a chemical solution container filled with a radioactive contrast medium to a medical facility is being established. Such a chemical solution container is formed of a cylindrical container body made of glass, and opened at its one end, and the opening of the container body is sealed by an elastic member made of silicone rubber or the like. The container body is placed inside a shield cover made of lead (or another radiation shielding material such as tungsten) and opened at its one end. A shield cap made of lead (or another radiation shielding material such as tungsten) is removably placed on the opening in the shield cover.

To fill the chemical solution syringe with the chemical solution from the chemical solution container, for example, a sharp needle member is put on the chemical solution syringe, and the shield cap is detached from the shield cover for the chemical solution container. Then, the needle member of the chemical solution syringe is inserted into the exposed elastic member, to absorb the contrast medium to the chemical solution syringe from the chemical solution container.

The needle member of the chemical solution syringe is replaced with the extension tube, which is then connected to the patient. The chemical solution syringe is mounted on the chemical solution injector, and the radioactive contrast medium is injected into the patient from the syringe, by the chemical solution injector.

Chemical solution injectors of the type described above have been invented and applied by the present applicant and the like (see, for example, patent documents 1 to 5 below).
Patent document 1: Japanese Patent Laid-Open No. 2002-11096;
Patent document 2: Japanese Patent Laid-Open No. 2002-102343;
Patent document 3: Japanese Patent Laid-Open No. 2002-210007;
Patent document 4: US6767319B2;
Patent document 5: US2003/0216609A1

The chemical solution container, which contains the radioactive contrast medium as described above, is wholly shielded by the shield cover and the shield cap made of lead or the like, to prevent the emission of the radiation of the contrast medium. However, when the contrast medium is absorbed from the chemical solution container into the chemical solution syringe, which is then mounted on the chemical solution injector, the operator is exposed to the radiation in that process.

To solve the abovementioned problem, the present applicant has proposed an imaging diagnostic system in which a radioactive contrast medium can be directly injected into a patient from a chemical solution container, and applied the system as Japanese Patent No. 2004-182475. In the imaging diagnostic system, the patient is connected to the chemical solution container through a chemical solution injection tube, the chemical solution container is connected to solution container via a liquid supply tube, and a chemical solution in a liquid container is pressed into the chemical solution container to inject the chemical solution into the patient from the chemical solution container.

In the imaging diagnostic system applied as described above, the distal end of the liquid supply tube is formed as a short needle member, and the proximal end of the chemical solution injection tube is formed as a long needle member. These short needle member and long needle member can be inserted into an elastic member of the chemical solution container to connect the liquid supply tube and the chemical solution injection tube to the chemical solution container without manually opening the elastic member of the chemical solution container.

In the imaging diagnostic system, however, the operator manually inserts the short needle member and the long needle member into the elastic member of the chemical solution container, so that the hands and fingers of the operator are exposed to radiation. In addition, after the abovementioned insertion is completed, the radiation may be transmitted through the elastic member of the chemical solution container during the injection work and then emitted into the environment.

### Disclosure of the Invention

The present invention has been made in view of the abovementioned problems, and it is an object thereof to provide a tube connection apparatus which minimizes radiation exposure of an operator when a liquid supply tube and a chemical solution injection tube are connected to an elastic member of a chemical solution container which contains a radioactive contrast medium.

The tube connection apparatus according to the present invention connects a tube member to a chemical solution container, which contains a chemical solution, an opening of a container body being sealed by an elastic member. The tube connection apparatus include:
a container holding mechanism for holding the chemical solution container removably, with the opening located above;
a short tubular needle member having a proximal end and a distal end, the proximal end being connected removably to a liquid supply tube in which a liquid with a specific gravity lower than that of the chemical solution flows, the distal end penetrates the elastic member;
a long tubular needle member having a proximal end and a distal end, the proximal end being connected removably to a chemical solution injection tube in which the chemical solution flows, the distal end penetrates the elastic member;
a holder member for holding the short needle member and the long needle member, with their proximal ends located above and their distal ends located below;
a holder moving mechanism for moving the short needle member and the long needle member vertically, together with the holder member between a retraction position above and an insertion position below; and
a container moving mechanism for supporting the chemical solution container together with the container holding mechanism to be movable horizontally, between an insertion position where the chemical solution container faces the short needle member and the long needle member from below at a insertion position and a retraction position where the chemical solution container does not face them.

In the tube connection apparatus according to the present invention, for example, while the short needle member and the long needle member are positioned at the retraction position by the holder moving mechanism, an operator manually places the chemical solution container on the container holding mechanism at the retraction position, and the chemical solution container is moved together with the container holding mechanism to the insertion position. In such a state, when the short needle member and the long needle member are moved to the insertion position, by the holder moving mechanism, the needle members penetrates the elastic member of the chemical solution container, and then the liquid supply tube and the chemical solution injection tube are connected to the chemical solution container.

Various means referred to in the present invention may be arranged to perform their functions, and may comprise dedicated hardware for performing a given function, an imaging diagnostic system whose given function is given by a computer program, a given function performed in an imaging diagnostic system according to a computer program, or a combination thereof. Various components referred to in the present invention do not need to be a separate entity. A plurality of means may be constructed as one member, a certain means may be part of another means, or a certain means may have a portion overlapping a portion of another means.

### Effect of the Invention

In the tube connection apparatus according to the present invention, the chemical solution container put on the chemical holding mechanism at the retraction position is moved to the insertion position by the container moving mechanism, and the short needle member and the long needle member are moved to the insertion position from the retraction position by the holder moving mechanism, thereby inserting the short needle member and the long needle member into the elastic member of the chemical solution container without requiring manual operation of the operator. Thus, the liquid supply tube and the chemical solution injection tube can be connected to the chemical solution container with minimized radiation exposure to the operator.

### Brief Description of the Drawings

Fig. 1 is a perspective view showing the outer appearance of a tube connection apparatus of an imaging diagnostic system according to an embodiment of the present invention;
Fig. 2 is a schematic diagram showing the whole imaging diagnostic system;
Fig. 3 is a perspective view showing the outer appearance of a chemical solution injector;
Fig. 4 is a perspective view showing how to mount a chemical solution syringe on an injection execution head of the chemical solution injector;
Fig. 5 is a schematic block diagram showing the circuit structure of the imaging diagnostic system;
Fig. 6 shows steps of the operation of the tube connection apparatus;
Fig. 7 is a perspective view showing the outer appearance of a tube connection apparatus of a first modification;
Fig. 8 is a perspective view showing the outer appearance of a tube connection apparatus of a second modification;
Fig. 9 is a perspective view showing the outer appearance of a specific example of the tube connection apparatus;
Fig. 10 is a perspective vies showing the outer appearance of a specific example of a holder member and short/long needle members;
Fig. 11 is a perspective view showing the outer appearance of a tube connection apparatus of a third modification;
Fig. 12 is a perspective view showing the internal structure of the tube connection apparatus;
Fig. 13 is a schematic block diagram showing the circuit structure of the imaging diagnostic system;
Fig. 14 shows steps of the operation of the tube connection apparatus;
Fig. 15 is a perspective view showing the internal structure of a tube connection apparatus of a fourth modification; and
Fig. 16 is a perspective view showing the internal structure of a tube connection apparatus of a fifth modification.

### Description of Reference Numerals

- 100: CHEMICAL SOLUTION INJECTOR
- 104: LIQUID CRYSTAL DISPLAY SERVING AS IMAGE DISPLAY MEANS
- 116: PISTON DRIVING MECHANISM SERVING AS LIQUID PRESSING MECHANISM
- 140: COMPUTER UNIT SERVING AS VARIOUS MEANS
- 120: LIQUID SUPPLY TUBE
- 130: CHEMICAL SOLUTION INJECTION TUBE
- 200: CHEMICAL SOLUTION SYRINGE SERVING AS LIQUID CONTAINER
- 210: CYLINDER MEMBER
- 220: PISTON MEMBER
- 300, 600, 700, 800, 900, 950, 960: TUBE CONNECTION APPARATUS
- 310, 910: CONTAINER MOVING MECHANISM
- 312: RETRACTION POSITION SENSOR SERVING AS POSITION SENSING MEANS
- 313: INSERTION POSITION SENSOR SERVING AS POSITION SENSING MEANS
- 315, 920, 961: CONTAINER HOLDING MECHANISM
- 320: HOLDER MOVING MECHANISM
- 330: SHIELD MOVING MECHANISM
- 335: SHIELD MEMBER
- 340: HOLDER MEMBER
- 341: SHORT NEEDLE MEMBER
- 342: LONG NEEDLE MEMBE
- 350,940: OPERATION CONTROL CIRCUIT SERVING AS OPERATION CONTROL MEANS
- 400: CHEMICAL SOLUTION CONTAINER
- 401: CONTAINER BODY
- 402: ELASTIC MEMBER
- 411: SHIELD COVER
- 412: SHIELD CAP
- 500: PET APPARATUS SERVING AS IMAGING DIAGNOSTIC APPARATUS
- 501: IMAGING DIAGNOSTIC UNIT SERVING AS IMAGING MEANS
- 502: IMAGING CONTROL UNIT SERVING AS VARIOUS MEANS
- 710: CCD CAMERA SERVING AS INSERTION IMAGING MEANS
- 921: MOVING GUIDE KNOB
- 923, 924: PLACEMENT LOCK MECHANISM
- 925: LOCK RELEASE KNOB
- 926: CONVEX PORTION SERVING AS LOCK RELEASE MECHANISM
- 930: CONTAINER ASCENT/DESCENT MECHANISM
- 951 1: CONTAINER ADAPTER

### Best Mode for Carrying Out the Invention

### [Configuration of Embodiment]

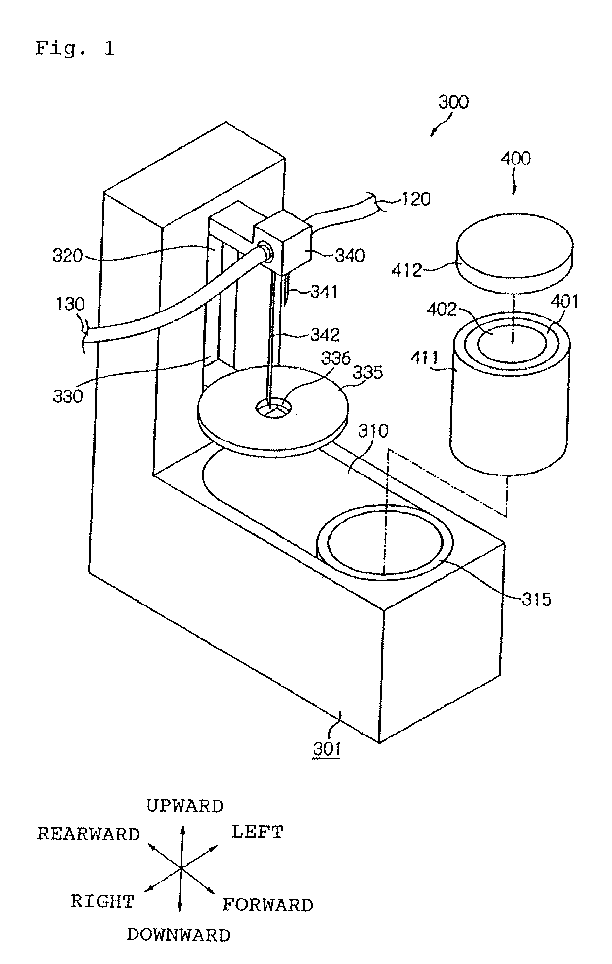

An embodiment of the present invention will hereinafter be described with reference to Figs. 1 to 16. As shown in Figs. 1 to 5,an imaging diagnostic system 1000 of the present embodiment has a chemical solution injector 100, a chemical solution syringe 200 serving as a liquid container, a tube connection apparatus 300, a chemical solution container 400, and a PET apparatus 500 serving as an imaging diagnostic apparatus. The chemical solution injector 100 injects a radioisotopic chemical solution as a radioactive contrast medium, into a patient (not shown) from chemical solution container 400. The PET apparatus 500 shoots diagnostic images of the patient.

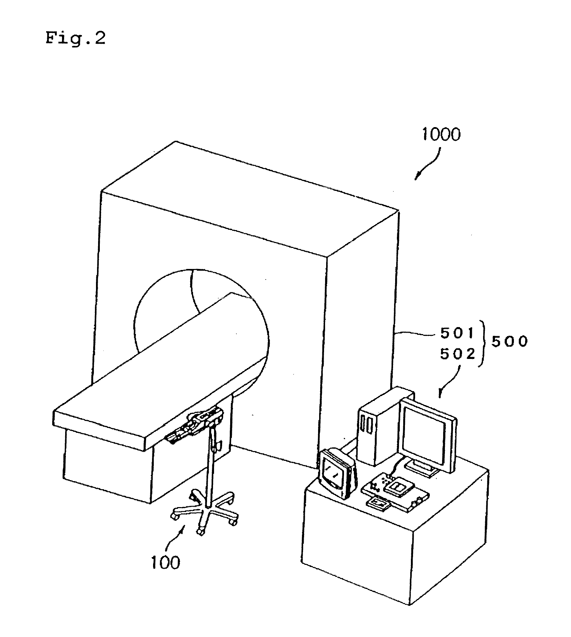

As shown in Fig. 2, the PET apparatus 500 includes an imaging diagnostic unit 501 serving as an imaging means and an imaging control unit 502,where the imaging diagnostic unit 501 and imaging control unit 502 are wire-connected. The imaging diagnostic unit 501 shoots diagnostic images of the patient. The imaging control unit 502 controls the operation of imaging diagnostic unit 501.

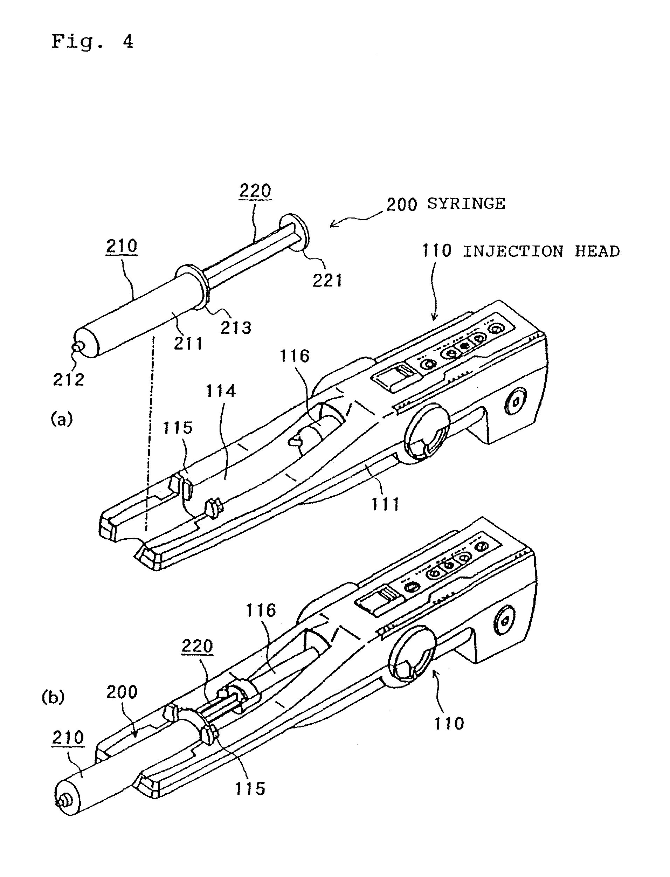

As shown in Fig. 4, the chemical solution syringe 200 comprises a cylinder member 210 and a piston member 220, wherein piston member 220 is slidably inserted into the cylinder member 210. The cylinder member 210 has a cylindrical transparent hollow cylinder body 211, which has a conduit 212 formed at the closed distal end surface.

The proximal end of the cylinder body 211 of cylinder member 210 is opened, and the piston member 220 is inserted from this opening into the cylinder body 211. The cylinder member 210 has a cylinder flange 213 formed in the outer circumference of the proximal end, and the piston member 220 has piston flange 221 formed in the outer circumference of the proximal end.

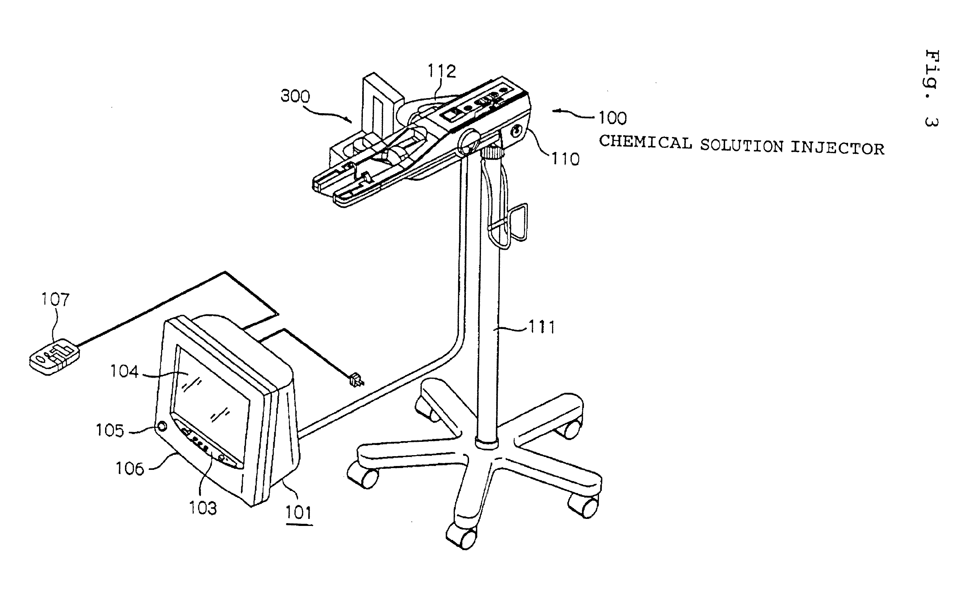

As shown in Fig. 3, a chemical solution injector 100 of the embodiment has an injection control unit 101and an injection execution head 110,wire-connected each other via a communication cable 102. The injection execution head 110 drives the chemical solution syringe 200 mounted thereon, to inject a chemical solution therefrom into a patient. The injection control unit 101 controls the operation of injection execution head 110.

The injection execution head 110 is attached to the upper end of a caster stand 111A by movable arm 112. As shown in Fig. 4, a head body 113 of the injection head 110 has a concave portion 114, formed as a semi-cylindrical groove in the upper surface for removably mounting chemical solution syringe 200. A cylinder holding mechanism 115 is formed in the forward section of concave portion 114, for removably holding cylinder flange 211 of chemical solution syringe 200. The piston driving mechanism 116 is placed as a liquid pressing mechanism, in the rearward section of concave portion 114, for holding and sliding piston flange 221.

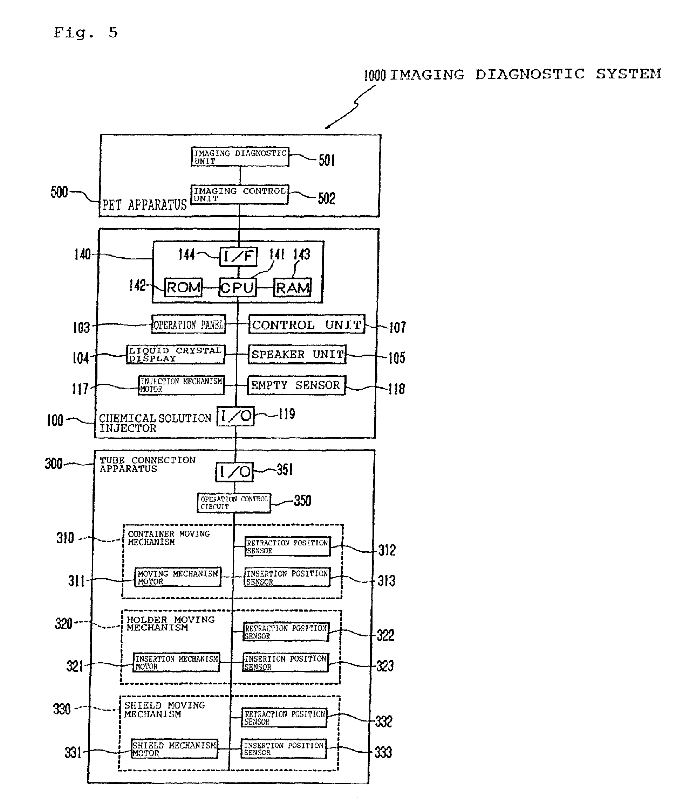

The piston driving mechanism 116 has an injection mechanism motor 117 (for example, Direct Current motor) as a driving source, which is formed of a as shown in Fig. 5, and a slides piston member 220 through a screw mechanism (not shown) or the like. An empty sensor 118 is also provided with the piston driving mechanism 116, it detects the completion of injection in chemical solution syringe 200 by detecting the slide position of piston driving mechanism 116.

An injection control unit 101 contains a computer unit 140 (shown in Fig. 5), and is also wire-connected to an imaging control unit 502 of the PET apparatus 500. As shown in Fig. 3, the injection control unit 101 has a operation panel 103, a liquid crystal display 104 serving as a data display means, and a speaker unit 105, all of which are disposed on the front face of unit housing 106. The injection control unit 101 is wire-connected to a controller unit 107,as a separate component.

As shown in Fig. 5, the chemical solution injector 100 of this embodiment is connected to the unit 140.The abovementioned various devices are connected to the computer unit 140, which integrates and controls those various devices. Computer unit 140 is provided of a so-called one-chip microcomputer, has hardware such as CPU (Central Processing Unit) 141, ROM (Read Only Memory) 142, RAM (Random Access Memory) 143, I/F (Interface) 144 and the like.

As shown in Fig. 3, in the imaging diagnostic system 1000 of the embodiment, a tube connection apparatus 300 is also attached to the upper end of the caster stand 111A of chemical solution injector 100, by movable arm 112 together with injection execution head 110. The chemical solution container 400 is removably put on the tube connection apparatus 300.

The chemical solution container 400 has cylindrical container body 401, made of glass and opened at its upper surface. The opening of the container body 401 is sealed by an elastic body 402, which is made of silicone rubber or the like. The container body 401 is inserted into a cylindrical shield cover 411.The shield cover 411 is made of tungsten, and opened at its upper surface. A shield cap 412 made of tungsten is removably put on the opening at the upper surface of shield cover 411.

In imaging diagnostic system 1000 of the embodiment, the chemical solution syringe 200 is filled with a physiological saline, and the chemical solution container 400 contains a radioisotopic solution as a radioactive contrast medium. The contrast medium is a liquid, with a specific gravity sufficiently higher than that of the physiological saline. The chemical solution container 400 contains the contrast medium of the quantity to be injected into a patient. The chemical solution syringe 200 contains the physiological saline of the quantity equal to, or larger than the same quantity as that of the contrast medium added to the quantity to be injected into the patient.

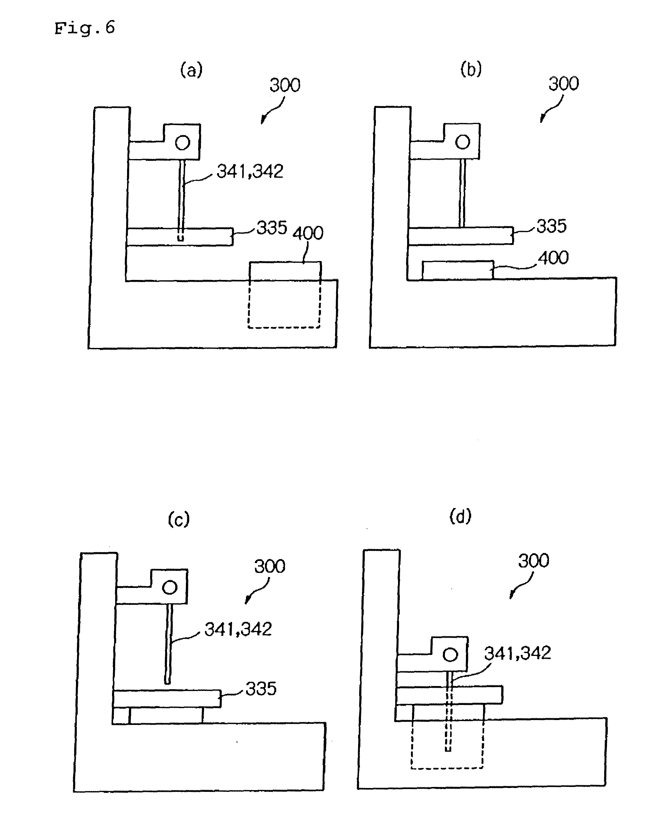

As shown in Figs. 1 and 6, the tube connection apparatus 300 of the embodiment has a L-shaped connection apparatus body 301, which includes a container moving mechanism 310 in a lower portion elongated in the forward-and-rearward direction. The container moving mechanism 310 shown in Fig. 5 includes a slider mechanism (not shown), includes a guide rail, a screw mechanism and the like, a moving mechanism motor 311 serving as a driving source (for example, DC motor or the like), and a retraction/insertion position sensors 312, 313 serving as position detecting means. The Container moving mechanism 310 moves a container holding mechanism 315 forward and rearward.

The container holding mechanism 315 is formed in a cylindrical hollow shape, having an opened upper surface and a closed lower surface. The chemical solution container 400 is put on it, with its opening is located at the top. The container moving mechanism 310 moves the container holding mechanism 315 between a retraction position at the front end of the movable range and an insertion position at the rear end of the movable range, by using the driving force of moving mechanism motor 311. The container moving mechanism 310 detects the placement of container holding mechanism 315 at the retraction position by retraction position sensor 312, and the placement of container holding mechanism 315 at the insertion position by an insertion position sensor 313.

A holder moving mechanism 320 and a shield moving mechanism 330 are arranged in the upper portion elongated in the up-and-down direction of the L-shaped connection apparatus body 301. The holder moving mechanism 320 supports a short/long needle members, movably in the up-and-down direction. The shield moving mechanism 330 supports the shield member, and moves it in the up-and-down direction.

More specifically, the holder moving mechanism 320 has a slider mechanism (not shown), a insertion mechanism motor 321, a retraction position sensor 322, an insertion position sensor 323 and the like, and move the holder member 340 between a retraction position at the upper end of the movable range and an insertion position at the lower end of the movable range vertically, by using the driving force of an insertion mechanism motor 321.The holder moving mechanism 320 detects the placement of the holder member 340 at the retraction/insertion positions, by retraction/insertion position sensors 322, 323, respectively.

The Holder member 340 projects forward from the front surface of the upper portion of the L-shaped connection apparatus body 301. Each needle members 341, 342 is attached to the lower surface of holder member 340,and protrude downward. Each needle members 341, 342 is formed in a tubular shape having a sharp distal end located below. The members 341,342 are arranged in parallel on the left and right.

The holder member 340 has channels (not shown), communicated to the needles, to which an end of liquid supply tube 120 and an end of chemical solution injection tube 130 are removably connected, the left and right respectively. The holder member 340 is removably attached to holder moving mechanism 320.

The chemical solution syringe 200 is removably connected to the end of liquid supply tube 120, and the end of chemical solution injection tube 130 is connected to a blood vessel of a patient via a catheter (not shown) or the like. In this way, the chemical solution syringe 200 is connected to chemical solution container 400 via liquid supply tube 120 and short needle member 341, and the chemical solution container 400 is connected to the patient via long needle member 342 and chemical solution injection tube 130. The chemical solution injection tube 130 is provided with an air-bubble removing mechanism (not shown), which removes air bubbles from the contrast medium, and physiological saline flowing in chemical solution injection tube 130.

In tube connection apparatus 300 of the embodiment, the respective components are sized appropriately. When the short/long needle members 341, 342 are moved to the retraction position, each needle members 341, 342 do not obstruct the chemical solution container 400.

In the standby state, where the holder moving mechanism 320 moves the short/long needle members 341, 342 at the retraction position, and the container moving mechanism 310 moves the chemical solution container 400 to the insertion position, the lower ends of each short/long needle members 341, 342, face the center of the upper surface of the chemical solution container 400 from above. In the insertion state, where the container moving mechanism 310 places chemical solution container 400 at the insertion position, and the chemical solution container 400 and short/long needle members 341, 342 are moved at the insertion positions, the distal end of short the needle member 341 is located in an upper portion inside chemical solution container 400, whereas the distal end of long needle member 342 is located in a lower portion inside of the chemical solution container 400.

Shield moving mechanism 330 has a slider mechanism (not shown), a shield mechanism motor 331, a retraction position sensor 332, a insertion position sensor 333 and the like, and moves the shield member 335 between a retraction position at the upper end of the movable range and an insertion position at the lower end of the movable range vertically, by using the driving force of the insertion mechanism motor 331.The moving mechanism 330 detects the placement of the shield member 335 at the retraction/insertion positions by retraction/insertion position sensors 332, 333, respectively.

Shield member 335 is formed in a disc shape and made of tungsten, and has an opening 336 formed at the center, through which the short/long needle members 341, 342 can be inserted. When the shield member 335 is placed at the retraction position by shield moving mechanism 330, the shield member 335 does not obstruct the chemical solution container 400, which is moved forward and rearward by container moving mechanism 310.

In the standby state, where the shield moving mechanism 330 places the shield member 335 at the retraction position, and the container moving mechanism 310 places the chemical solution container 400 at the insertion position, the shield member 335 faces the opening in the upper surface of the chemical solution container 400 from above. In the shield state, where container moving mechanism 310 places chemical solution container 400 at the insertion position and shield member 335 is placed at the insertion position, the opening in the upper surface of chemical solution container 400 is shielded by shield member 335.

For example, as shown in Fig. 5, tube connection apparatus 300 of the embodiment has an operation control circuit 350 formed of an ASIC (Application Specific Integrated Circuit) as an operation control means. Various mechanism motors 311, 321, and 331, and various position sensors 312, 313, ... , 333 are connected to operation control circuit 350. Operation control circuit 350 controls the driving of various mechanism motor 311 and the like, in accordance with the detection results of various position sensor 312 and the like to integrate and control container moving mechanism 310, holder moving mechanism 320, and shield moving mechanism 330.

Thus, the operation control circuit 350 sequentially produces following states in a forward and rearward order: a retraction state as shown in Fig. 6(a) in which chemical solution container 400, shield member 335, and short/long needle members 341, 342 are placed at the retraction positions, a standby state as shown in Fig. 6(b) in which chemical solution container 400 is located at the insertion position, and shield member 335 and short/long needle members 341, 342 are located at the retraction positions, a shield state as shown in Fig. 6(c) in which chemical solution container 400 and shield member 335 are placed at the insertion positions and short/long needle members 341, 342 are placed at the retraction position, and an insertion state as shown in Fig. 6(d) in which chemical solution container 400, shield member 335, and short/long needle members 341, 342 are located at the insertion positions.

An I/O (Input/Output) port 351 is connected to operation control circuit 350 of the tube connection apparatus 300. An I/O port 119 corresponding to a data communicating means is connected to computer unit 140 of chemical solution injector 100. The chemical solution injector 100 is wire-connected to tube connection apparatus 300 through I/O ports 351 and 119, the operations of tube connection apparatus 300, chemical solution injector 100, and PET apparatus 500 are mutually associated in imaging diagnostic system 1000 of the embodiment.

Thus, the computer unit 140 of chemical solution injector 100 has an appropriate computer program installed as firmware or the like in an information storage medium such as ROM 142, and CPU 141 executes various types of processing in accordance with the computer program.

Computer unit 140 performs various types of processing, in accordance with the computer program installed as described above, to allow chemical solution injector 100 of the embodiment to logically have various means such as a press control means, a time detecting means, a data transmitting means and the like. More specifically, the press control means of chemical solution injector 100 corresponds to the function of computer unit 140, which controls the operation of injection mechanism motor 117 in accordance with the communication result with tube connection apparatus 300, and allows the piston driving mechanism 116 to be operative only when the tube connection apparatus 300 is in the insertion state.

The time detecting means corresponds to the function of computer unit 140, which counts a clock signal based on a signal output from empty sensor 118, and detects the lapse of a given time period when empty sensor 118 detects the completion of injection. The data transmitting means corresponds to the function of computer unit 140 which transmits given data to communication I/F 144 and transmits the detected lapse of time period to PET apparatus 500.

Similarly, imaging control unit 502 performs various types of processing in accordance with the computer program to allow PET apparatus 500 to logically have various means such as a data receiving means, an imaging control means, and the like. The data receiving means receives the lapse of time period from chemical solution injector 100. The imaging control means causes imaging diagnostic unit 501 to start imaging when the lapse of time period is received.

### [Operation of the Embodiment]

When the imaging diagnostic system 1000 of the embodiment is used in the abovementioned structure, a chemical solution syringe 200 filled with the physiological saline and a chemical solution container 400 filled with the contrast medium are prepared, and each is mounted on chemical solution injector 100 and tube connection apparatus 300, respectively.

In this case, for example, the chemical solution syringe 200 is connected to holder member 340 via the liquid supply tube 120, and chemical solution syringe 200 is mounted on injection execution head 110 of chemical solution injector 100. Then, cylinder member 210 of chemical solution syringe 200 is held by cylinder holding mechanism 115 and piston member 220 is held by piston driving mechanism 116, so that chemical solution injector 100 can press piston member 220 into cylinder member 210 of chemical solution syringe 200.

In the initial state, the chemical solution injector 100 controls piston driving mechanism 116 to be inoperative, therefore the piston driving mechanism 116 is not operated accidentally. When liquid supply tube 120 is connected to holder member 340 of tube connection apparatus 300 as described above, chemical solution injection tube 130 is connected to holder member 340, for example.

As shown in Figs. 1 and 6(a), in tube connection apparatus 300, the container holding mechanism 315, shield member 335, and short/long needle members 341, 342 are placed at the retraction positions in the retraction state as the initial state, thus the short/long members 341, 342 do not obstruct the container holding mechanism 315.

Operator detaches the shield cap 412 from the shield cover 411 of chemical solution container 400, and puts the container 400 on container holding mechanism 315 from above. In this state, an operator makes entry of "start connection" to operate the operation panel 103 of chemical solution injector 100. The entered data is transmitted to tube connection apparatus 300 from chemical solution injector 100.

Then, in tube connection apparatus 300, as shown in Fig. 6(b), the chemical solution container 400 is moved to the insertion position at the rear end by container moving mechanism 310. Next, as shown in Fig. 6(c), the shield member 335 is moved to the insertion position at the lower end from the retraction position at the upper end by shield moving mechanism 330. And then, the opening of the chemical solution container 400 is covered by shield member 335, the chemical solution container 400 is held from above.

Next, as shown in Fig. 6(d), the short/long needle members 341, 342 are moved downwardly to the insertion position at the lower end from the retraction position at the upper end by holder moving mechanism 320, so that the short/long needle members 341, 342 are inserted into the elastic member 402 of chemical solution container 300 opening 336 of shield member 335. In this way, the distal end of short needle member 341 is located in the upper portion inside of the chemical solution container 400, and the distal end of long needle member 342 is located in the lower portion inside of the chemical solution container 400.

Then, the tube connection apparatus 300 transmits data representing "connection completed" to chemical solution injector 100. The chemical solution injector 100 outputs "connection completed" with display on liquid crystal display 104 of injection control unit 101,and the inoperative state of piston driving mechanism 116 is cleared.

In this state, for example, when the operator makes entry of "perform preparatory operation" to operation panel 103 of chemical solution injector 100, the piston driving mechanism 116 is driven for a given time. Then, a given quantity of the physiological saline is supplied to the chemical solution container 400 from chemical solution syringe 200 via liquid supply tube 120.Thus, the contrast medium is injected and filled into the chemical solution injection tube 130 from sealed chemical solution container 400.

After liquid supply/chemical solution injection tubes 120, 130 are filled with the physiological saline and contrast medium, respectively, and the air is removed therefrom in this manner, the distal end of chemical solution injection tube 130 is connected to a blood vessel of a patient. In this state, when the operator makes entry of "perform injection operation" to operation panel 103, for example, the piston driving mechanism 116 is driven until empty sensor 118 detects the end of injection.

Then, the physiological saline in chemical solution syringe 200 is supplied to chemical solution container 400 via liquid supply tube 120.

Since the physiological saline has a specific gravity lower than that of the contrast medium, the contrast medium is located below and the physiological saline is located above inside of the chemical solution container 400. The contrast medium is injected into the patient via chemical solution injection tube 130 from long needle member 342 having the distal end placed below inside of the chemical solution container 400.

In chemical solution injector 100 of the embodiment, when the quantities of chemical solution syringe 200 and chemical solution container 400 are appropriately adjusted, all of the contrast medium is firstly injected into the patient from chemical solution container 400, and then the physiological saline supplied into chemical solution container 400 with pressure from chemical solution syringe 200 is then injected into the patient.

The physiological saline pushes the contrast medium, thus the contrast medium reaches a body part of the patient to be imaged by PET apparatus 500. The contrast medium reaching the body part to be imaged is more highly absorbed by cancer cells than by normal cells, but the absorption requires a given time period.

The chemical solution injector 100 of the embodiment count a given time for the cells to absorb the contrast medium after empty sensor 118 detects the end of injection of the contrast medium as described above. When the lapse of the time period is detected, this data is transmitted to the PET apparatus 500, which then receives the lapse data and takes a diagnostic image of the patient by imaging diagnostic unit 501.

Upon completion of the taking of the diagnostic image, the PET apparatus 500 transmits data representing the completion to chemical solution injector 100. Chemical solution injector 100 receives the "taking completed" and transmits "release connection" to tube connection apparatus 300. Upon reception of that, tube connection apparatus 300 causes holder moving mechanism 320 to move short/long needle members 341, 342 to the retraction position at the upper end from the insertion position at the lower end as shown in Fig. 6(c).

As shown Fig. 6(b), shield moving mechanism 330 moves shield member 335 to the retraction position at the upper end from the insertion position at the lower end. As shown in Figs. 1 and 6(a), the container moving mechanism 310 moves chemical solution container 400 to the retraction position at the front end from the insertion position at the rear end. The operator removes chemical solution container 400 from container holding mechanism 315, and for example, takes container body 401 out of shield cover 411, and discards it. Shield cover 411 and shield cap 412 may be reused later.

### [Effect of the Embodiment]

According to the imaging diagnostic system 1000 of the embodiment, the physiological saline in chemical solution syringe 200 is supplied to chemical solution container 400 via liquid supply tube 120 with pressure by piston driving mechanism 116, to inject the contrast medium in chemical solution container 400 into the patient from chemical solution injection tube 130.
Therefore the contrast medium can be injected directly into the patient from chemical solution container 400, without using a refill-type chemical solution syringe. Therefore the operator does not need to fill the contrast medium into the chemical solution syringe from chemical solution container 400, or to handle the syringe.

Furthermore, the short/long needle members 341, 342 are moved by the tube connection apparatus 300, the operator does not need to perform manually the insertion. After the operator puts chemical solution container 400 with shield cap 412 removed from shield cover 411 on tube connection apparatus 300, the operator does not need to handle chemical solution container 400 until the end of injection. The radiation exposure to the operator can be reduced favorably.

Furthermore, the container holding mechanism 315, on which chemical solution container 400, is placed is disposed at the retraction position at the front end where shield member 335 or the like does not interfere in the initial state, therefore the operator can immediately put chemical solution container 400 on tube connection apparatus 300. This can reduce the time for the operator to handle chemical solution container 400 from which shield cap 412 is detached and which emits radiation.

Furthermore, according to the tube connection apparatus 300 of the embodiment, first the shield member 335 covers the opening of the container 400, therefore it is possible to reduce the time for the operator to handle chemical solution container 400. In addition, the injection control unit 101 of chemical solution injector 100 can remotely operate the tube connection apparatus 300, the operator does not need to operate manually tube connection apparatus 300 on which chemical solution container 400 is put.

Furthermore, in tube connection, the shield member 335 closes the opening in chemical solution container 400 and holds chemical solution container 400 from above. Thus, short/long needle members 341, 342 can be inserted stably into elastic member 402 of chemical solution container 400, and it is possible to prevent elastic member 402 from coming off chemical solution container 400 due to the pressure of the injected physiological saline.

Furthermore, the short/long needle members 341, 342 are inserted into the elastic member 402 through the opening336 with a small diameter in shield member 335, the opening 336 can guide the short/long needle members 341, 342. Therefore, the short/long needle members 341, 342 can be inserted into elastic member 402 more stably.

Furthermore, the container moving mechanism 310 moves the chemical solution container 400 horizontally, and, the holder moving mechanism 320 and shield moving mechanism 330 move the short/long needle members 341, 342 and the shield member 335vertically,respectively. Therefore, the moving mechanisms 310, 320, and 330 operate simply and have simple structures with favorable durability and reliability

Since the horizontal movement of chemical solution container 400 is combined with the vertical movements of short/long needle members 341, 342 and shield member 335 as described above, the moving areas of the respective components can be minimized to reduce the whole size and weight of tube connection apparatus 300. Especially, since the holder moving mechanism 320 can share a guide rail or the like with the shield moving mechanism 330, the mechanisms can be further simplified to realize a reduction in size and weight.

Tube connection apparatus 300 of the embodiment automatically and sequentially produces the retraction state in which chemical solution container 400, shield member 335, and short/long needle members 341, 342 are placed at the retraction positions, the standby state in which chemical solution container 400 is located at the insertion position, and shield member 335 and short/long needle members 341, 342 are located at the retraction positions, the shield state in which chemical solution container 400 and shield member 335 are placed at the insertion positions and short/long needle members 341, 342 are placed at the retraction position, and the insertion state in which chemical solution container 400, shield member 335, and short/long needle members 341, 342 are located at the insertion positions.

Thus, the operator does not need to manually operate respective moving mechanisms 310, 320, and 330 individually, and short/long needle members 341, 342 can be inserted appropriately into elastic member 402 of chemical solution container 400. Since the tube connection apparatus 300 of the embodiment sequentially produces the abovementioned states in the reverse order, chemical solution container 400 can be easily and immediately detached after the completion of the injection operation.

Since the medium for pressing the contrast medium is realized by the physiological saline, the contrast medium can be pressed by the medium that causes no problem even when it is injected into the patient. The contrast medium has a specific gravity sufficiently higher than that of physiological saline, and the distal end of liquid supply tube 120 is located above inside chemical solution container 400 and the proximal end of chemical solution injection tube 130 is located below inside chemical solution container 400, so that only the contrast medium can be injected first into the patient and then the physiological saline can be injected readily.

Since short/long needle members 341, 342 to liquid supply/chemical solution injection tubes 120, 130 are held together by holder member 340, liquid supply/chemical solution injection tubes 120, 130 can be connected to chemical solution container 400 more easily, and the distal ends of short/long needle members 341, 342 can be easily and reliably disposed at appropriate positions inside chemical solution container 400.

Short/long needle members 341, 342 are formed integrally with holder member 340, and holder member 340 is removably put on holder moving mechanism 320. This allows simple replacement of holder member 340 and short/long needle members 341, 342 in contact with the physiological saline and contrast medium to keep tube connection apparatus 300 clean at all times without requiring special cleaning.

In imaging diagnostic system 1000 of the embodiment, tube connection apparatus 300 and chemical solution injector 100 are formed as the separate components, and it is essential only that chemical solution injector 100 can supply the physiological saline into tube connection apparatus 300 with pressure. For this reason, an existing product can be used as chemical solution injector 100 to form imaging diagnostic system 1000 of the embodiment easily.

In imaging diagnostic system 1000 of the embodiment, chemical solution injector 100 can control piston driving mechanism 116 to be operative only when tube connection apparatus 300 is in the insertion state. Chemical solution syringe 200 is not driven when liquid supply/chemical solution injection tubes 120, 130 are not connected to chemical solution container 400.

In imaging diagnostic system 1000 of the embodiment, when chemical solution injector 100 detects the lapse of the given time period from the end of the injection, PET apparatus 500 automatically starts shooting of images. It takes a given time period for the contrast medium for PET to be optimal for shooting of diagnostic images after it is injected into a patient, but the lapse of the time period can be automatically measured and diagnostic images can be taken in an optimal state.

### [Modifications of the Embodiment]

The present invention is not in any way limited to the abovementioned embodiment, but various changes and modifications may be made therein without departing from the scope of the invention. For example, in the above embodiment, the chemical solution injector 100 and the tube connection apparatus 300 are separate components, but they may be integrated into one component.

In the above embodiment, the container moving mechanism 310 of the tube connection apparatus 300 horizontally moves the chemical solution container 400 together by container holding mechanism 315 through the driving force of moving mechanism motor 311. For example, the operator may manually drive a container moving mechanism (not shown) with no driving source, to horizontally move chemical solution container 400 together with container holding mechanism 315 (not shown).

In this case, the radiation exposure to the operator is increased as compared with the tube connection apparatus 300 described above. However, the chemical solution container 400 from which shield cap 412 detached is expected to emit radiation upward. Therefore a manually operable portion may be formed in front or on the side of the container holding mechanism 315, to minimize the radiation exposure to the operator while chemical solution container 400 can be horizontally moved through manual operation.

In a tube connection apparatus (not shown) in which chemical solution container 400 is horizontally moved with manual operation as described above, it is preferable that retraction/insertion position sensors 312, 313 are placed in the container moving mechanism, holder moving mechanism 320 and shield moving mechanism 330 are forcedly placed at the retraction positions when chemical solution container 400 is disposed at the retraction position, and holder moving mechanism 320 and shield moving mechanism 330 are operative only when chemical solution container 400 is placed at the insertion position.

In the tube connection apparatus in which chemical solution container 400 is horizontally moved with manual operation as described above, for example, it is possible to use a structure (not shown) in which the convex portion of container holding mechanism 315 is fitted into the concave portion of the container moving mechanism to hold chemical solution container 400 at the insertion position and the retraction position.

In the above embodiment, tube connection apparatus 300 covers the opening in chemical solution container 400 with shield member 335, but such shield member 335 may not be included. While shield member 335 is made of tungsten in the above embodiment, this may be made of lead, and container holding mechanism 315 may be made of tungsten or lead.

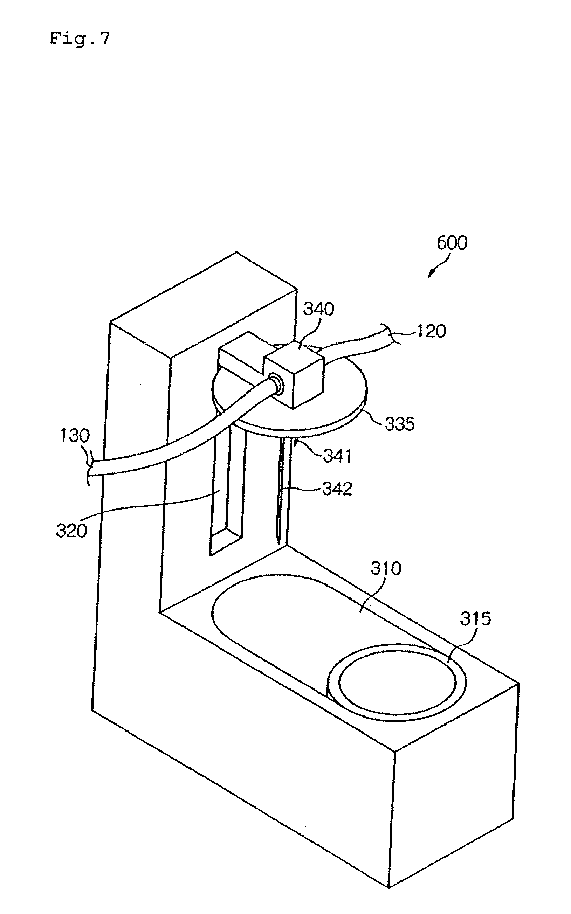

In the above embodiment, shield member 335 is moved by dedicated shield moving mechanism 330. For example, shield member 335 may be fixed at the position opposite to the opening in chemical solution container 400 located at the insertion position from above (not shown). Alternatively, as in tube connection apparatus 600 shown in Fig. 7, shield member 335 may be formed integrally with holder member 340 (not shown).

In this case, since the opening in chemical solution container 400 is not covered with shield member 335 before the insertion of short/long needle members 341, 342 into chemical solution container 400, the radiation exposure time of the operator is increased. However, dedicated shield moving mechanism 330 is not required and the structure of the tube connection apparatus can be simplified.

In the above embodiment, chemical solution injector 100 can be manually operated to control tube connection apparatus 300 remotely. For example, a dedicated operation panel (not shown) may be mounted on tube connection apparatus 300. When tube connection apparatus 300 is remotely controlled as described above, it is difficult to simply and immediately check whether or not short/long needle members 341, 342 are inserted appropriately into elastic member 402 without radiation exposure.

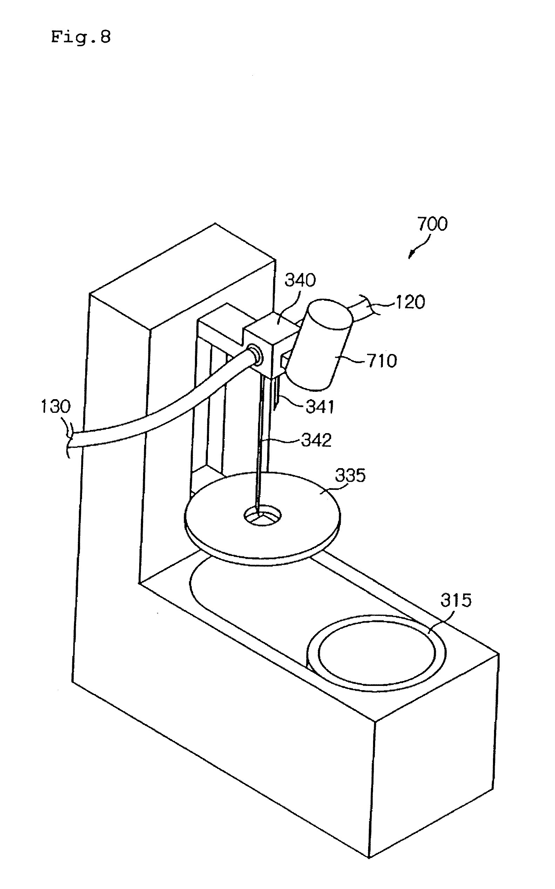

If this presents any problem, it is preferable to provide a CCD (Charge Coupled Device) camera 710 as an insertion imaging means on the front surface of holder member 340 or the like, as shown in tube connection apparatus 700 illustrated in Fig. 8. In this case, since the CCD camera 710 can shot the point where short/long needle members 341, 342 are inserted into the elastic member 402, the taken image can be output with display on liquid crystal display 104 of chemical solution injector 100.

When operation panel 103 of chemical solution injector 100 is manually operated to cause tube connection apparatus 700 to insert short/long needle members 341, 342 into elastic member 402, the insertion state is output with display on liquid crystal display 104 placed near operation panel 103 to allow the operator to simply check whether or not short/long needle members 341, 342 are appropriately inserted into elastic member 402 in real time without exposure to radiation.

In the above embodiment, liquid supply/chemical solution injection tubes 120, 130 are connected to chemical solution syringe 200 and chemical solution container 400, and then liquid supply/chemical solution injection tubes 120, 130 are filled with the contrast medium and physiological saline to eliminate the air. For example, liquid supply/chemical solution injection tubes 120, 130 may be filled with physiological saline or the like before tubes 120, 130 are connected to chemical solution syringe 200 and chemical solution container 400.

In the above embodiment, air bubbles mixed in the contrast medium are removed by the air-bubble removing mechanism. For example, it is possible that an air-bubble detection sensor (not shown) is placed downstream of the air-bubble removing mechanism, and if the air-bubble detection sensor detects any bubble, piston driving mechanism 116 is forcedly stopped. It is also possible that chemical solution injection tube 130 is blocked by a dedicated injection shield mechanism (not shown).

It is also possible that a switch detection sensor (not shown) is mounted on chemical solution injection tube 130 for detecting a switch from the radioactive contrast medium to the physiological saline based on the presence or absence of radiation, and piston driving mechanism 116 is forcedly stopped if that switch is detected in chemical solution injection tube 130.

In chemical solution injector 100 of the embodiment, the contrast medium in chemical solution container 400 is pushed by the physiological saline and thus injected into the patient, so that it is difficult to properly check whether or not all of the contrast medium in chemical solution container 400 is injected into the patient. If the switch detection sensor detects the switch from the contrast medium flowing in chemical solution injection tube 130 to the physiological saline, the operator can easily and reliably check whether or not all of the contrast medium in chemical solution container 400 is injected into the patient, and piston driving mechanism 116 can be stopped in a desired timing.

When the switch detection sensor detects the switch from the contrast medium flowing in chemical solution injection tube 130 to the physiological saline, piston driving mechanism 116 may be stopped after a given quantity of the physiological saline is supplied with pressure. In this case, since the given quantity of the physiological saline is injected into the patient following the contrast medium, the physiological saline can cause the contrast medium to reliably reach a body part to be imaged in the patient.

In the above embodiment, the volumes of chemical solution syringe 200 and chemical solution container 400 are appropriately adjusted, and all of the physiological saline in chemical solution syringe 200 is injected and causes the contrast medium to properly reach the body part to be imaged in the patient. For example, the volume of the physiological saline to be injected from chemical solution syringe 200 may be controlled in order to inject the exact quantity of the contrast medium into the body part to be imaged.

In this case, for example, it is preferable that data of a liquid quantity is stored for each body part to be imaged on computer unit 140 of injection control unit 101 of chemical solution injector 100, and when data of a body part to be imaged in a patient is entered on operation panel 103, the quantity of physiological saline to be injected is controlled in accordance with the body part to be imaged.

In the above embodiment, chemical solution injector 100 measures the given time period taken for the injected contrast medium to be absorbed by cells and transmits that data to PET apparatus 500. For example, it is possible that chemical solution injector 100 immediately transmits data representing the end of injection to PET apparatus 500 which then measures the given time period after the end of injection and then automatically starts imaging operation.

In the above embodiment, piston driving mechanism 116 supplies the physiological saline with pressure from chemical solution syringe 200 to chemical solution container 400 via liquid supply tube 120, to supply the contrast medium with pressure from chemical solution container 400 to the patient via chemical solution injection tube 130. If only the supply with pressure by piston driving mechanism 116 may cause backflow of the physiological saline or contrast medium, it is possible to insert one-way valve (not shown) on liquid supply tube 120 for regulating the flow of the physiological saline in one direction. Otherwise, it is possible to insert one-way valve (not shown) on chemical solution injection tube 130 for regulating the flow of contrast medium in one direction, for example.

In the above embodiment, the piston member is pressed into the cylinder member of the chemical solution syringe filled with the physiological saline in chemical solution injector 100 to supply the physiological saline with pressure to liquid supply tube 120 connected to the chemical solution syringe. The chemical solution injector may include a peristaltic finger mechanism or a rotary pump mechanism to sequentially press liquid supply tube 120 connected to the liquid container from the outside to supply the physiological saline with pressure (not shown).

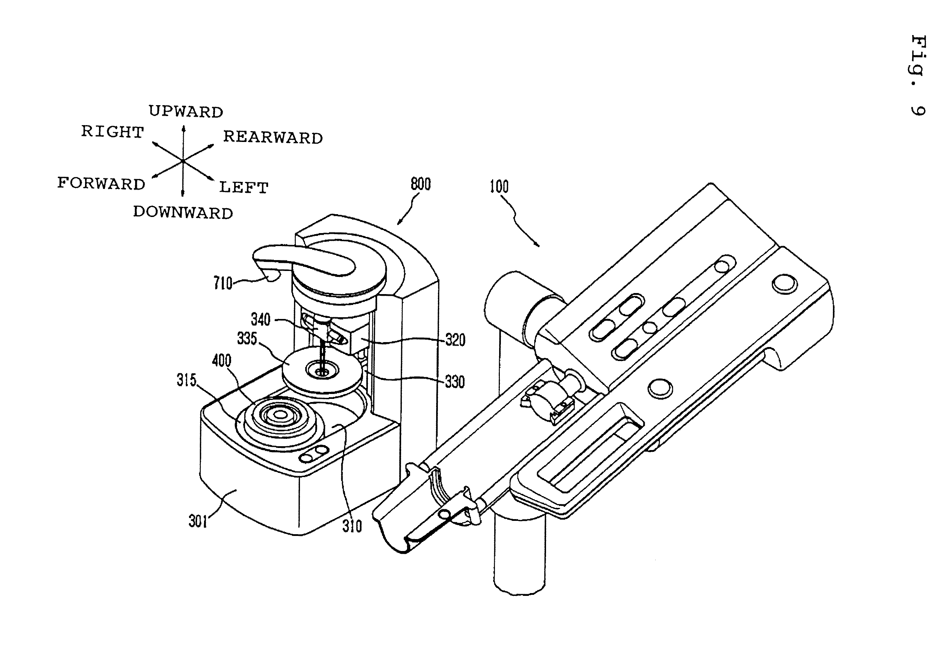

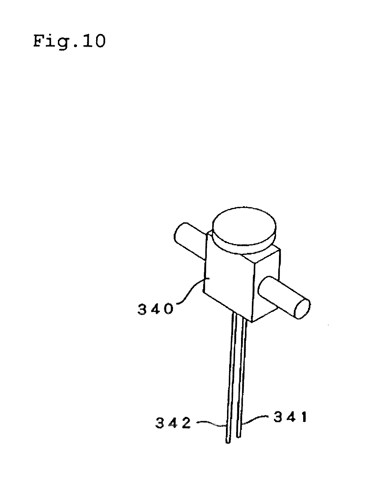

To simplify the description, the structure of tube connection apparatus 300 or the like has been schematically shown and explained in the above embodiment. The present inventor prototyped a tube connection apparatus. Prototyped tube connection apparatus 800 will hereinafter be described in brief with reference to Figs. 9 and 10.

Tube connection apparatus 800 basically has the structure similar to that of tube connection apparatus 700 described above. The tube connection apparatus 800 includes a connection apparatus body 301, a container moving mechanism 310, a container holding mechanism 315, a holder moving mechanism 320, a shield moving mechanism 330, a shield member 335, a holder member 340, short/long needle members 341, 342, and a CCD camera 710.

In this tube connection apparatus 800, the CCD camera 710 is put on the connection apparatus body 301, and is supported revolvably in the left-and-right direction, for example. The operation panel 810 is added on the left of the upper surface of connection apparatus body 301. By operating this operation panel 810 manually, the retraction state and the insertion state can be switched. In addition, as shown in Fig. 10, holder member 340 and short/long needle members 341, 342 are formed as a minimum unit structure. As shown in Fig. 9, short/long needle members 341, 342 are removably put on holder moving mechanism 320 together with holder member 340.

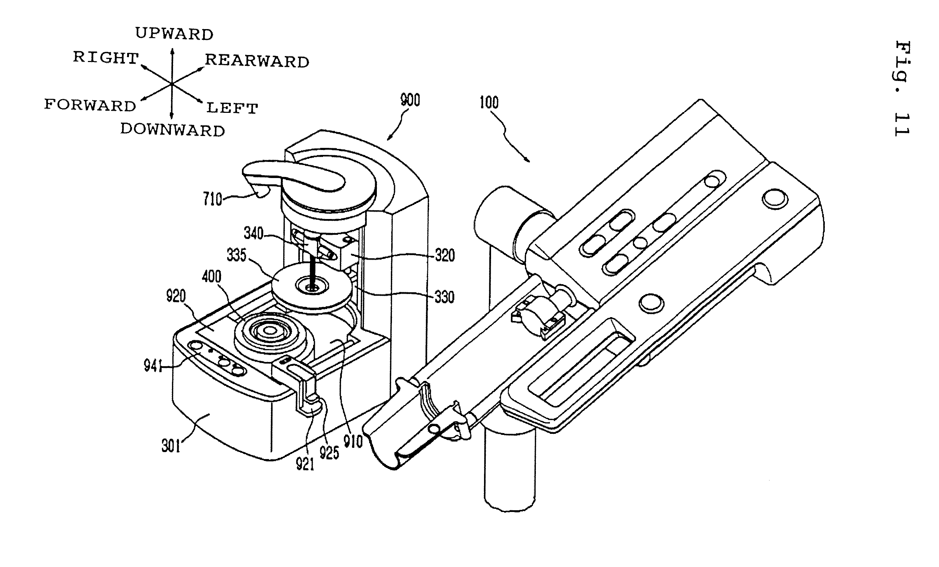

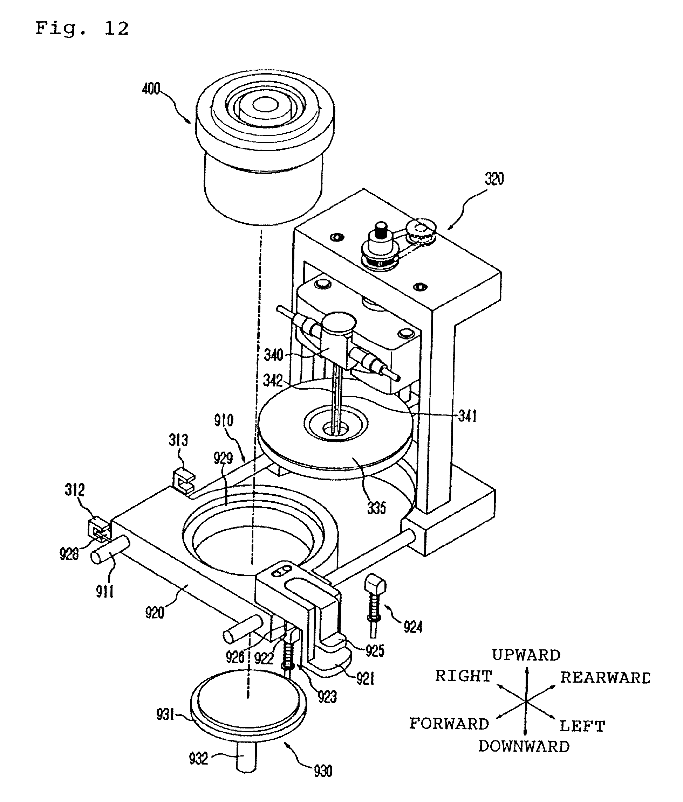

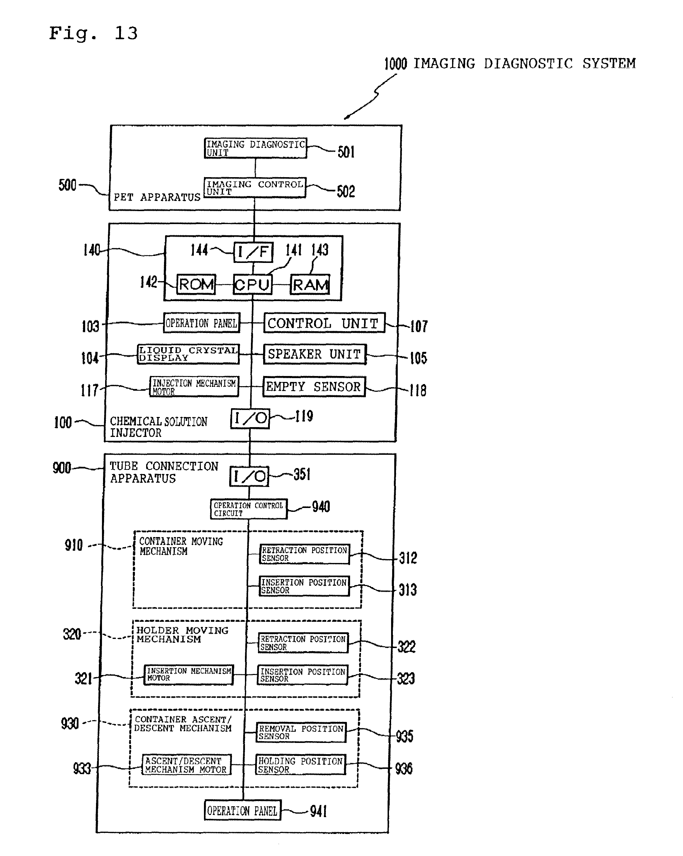

A second prototype of tube connection apparatus 900 will hereinafter be described in brief with reference to Figs. 11 to 14. As shown in Fig. 11, this tube connection apparatus 900 is basically provided to have a structure similar to that of tube connection apparatus 800. However, as shown in Fig. 12, a container moving mechanism 910 includes a pair of guide rails 911, and as shown in Fig. 13, the tube connection apparatus 900 includes no driving source such as moving mechanism 311.

The container holding mechanism 920 includes a moving guide knob 921 provided on the left, and can be pinched by fingers. Container holding mechanism 920 is moved manually forward and rearward together with moving guide knob 921. The Container holding mechanism 920 has a concave portion 922 in a lower surface on the left and a pair of placement lock mechanisms 923, 924 including an engagement hook and a coil spring, which elastically engages with concave portion 922,to lock container holding mechanism 920 to a retraction position and an insertion position.

The moving guide knob 921 is formed in a crank shape, which extends leftward, downward, and leftward from container holding mechanism 920 in order. A lock release knob 925 is attached to the vertical portion of it, slidabley. The lock release knob 925 is elastically urged upward by a coil spring (not shown) or the like, and has integrally formed convex portion 926 on the right end, which protrudes downward and serves as a lock release mechanism.

When the moving guide knob 921 and the lock release knob are pinched, the knob 925 is also displaced downward. Then, the convex portion 926 of lock release knob 925 presses downward the placement lock mechanisms 923, 924 engaging with concave portion 922 of container holding mechanism 920, therefore the lock of container holding mechanism 920 by placement lock mechanisms 923, 924 is released.

Container holding mechanism 920 has integrally formed flat-shaped convex portion 928 on the right end. A pair of photo sensors for optically sensing that convex portion 928 forms retraction/insertion position sensors 312, 313 for sensing container holding mechanism 920 at the retraction position and the insertion position. Container holding mechanism 920 holds chemical solution container 400 in opening 929 opened vertically. Container ascent/descent mechanism 930 is placed at the position facing from below opening 929 of container holding mechanism 920 at the retraction position.

As shown in Figs. 12 to 14, the container ascent/descent mechanism 930 includes a discoid holding table 931 for holding the chemical solution container 400 from below. The holding table 931 is supported by guide shaft 932 to be movable vertically. Since the ascent/descent mechanism motor 933 is connected to holding table 931 by a screw mechanism (not shown), the driving force of ascent/descent mechanism motor 933 vertically moves holding table 931.

The container ascent/descent mechanism 930 presses from below chemical solution container 400 placed in opening 929 of container holding mechanism 920 at the retraction position with holding table 931 which is movable vertically. This vertically moves chemical solution container 400 at the retraction position to a holding position where it is held by container holding mechanism 920 at the retraction position and to a removal position above the holding position.

The container ascent/descent mechanism 930 is provided with, for example, a removal position sensor 935 for detecting the placement of chemical solution container 400 at the removal position, and a holding position sensor 936 for detecting the placement at the holding position with a photo sensor (not shown) for optically detecting holding table 931 or the like.

The tube connection apparatus 900 includes a shield member 335, which is fixed at a position opposite to chemical solution container 400 placed at the insertion position from above. An operation panel 941 is also provided on the upper surface at the front end of a connection apparatus body 301, and is connected to a operation control circuit 940 serving as an operation control means.

The operation control circuit 940 controls the operation of the container ascent/descent mechanism 930 and the holder moving mechanism 320, based on the entry operation on operation panel 941 and the detection result of the retraction/insertion position sensors 312, 313. The operation control circuit 940 controls the container ascent/descent mechanism 930 to be operable only when the chemical solution container 400 is positioned at the retraction position, and the controls container ascent/descent mechanism 930 to be at the holding position when the chemical solution container 400 is not positioned at the retraction position. The operation control circuit 940 controls the holder moving mechanism 920 to be operable, only when the chemical solution container 400 is positioned at the insertion position, and controls the holder moving mechanism 920 to be at the retraction position when the chemical solution container 400 is not positioned at the insertion position.

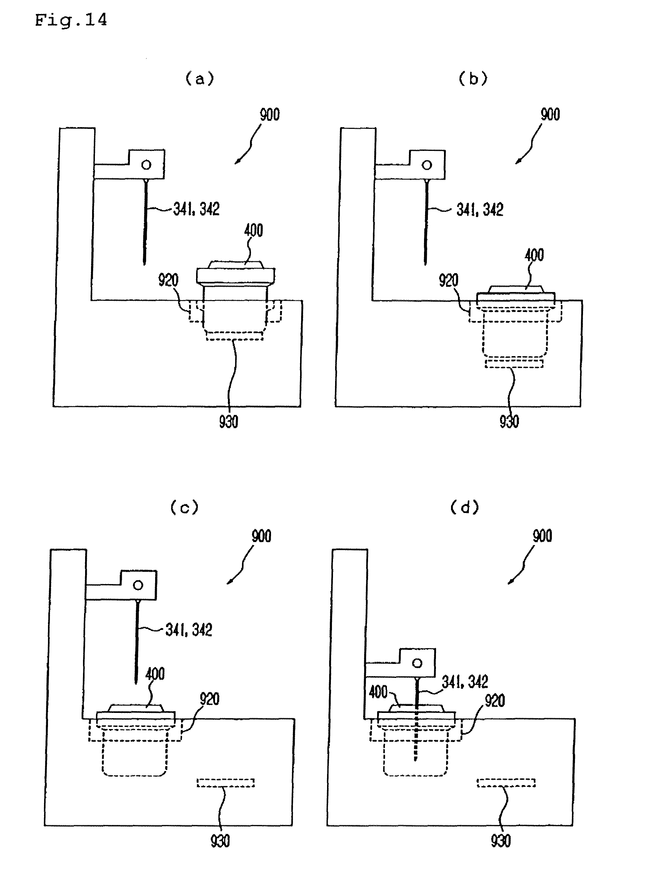

As shown in Fig. 14(a), in tube connection apparatus 900 of the abovementioned structure, container holding mechanism 920 is manually placed at the retraction position in the initial state, and container ascent/descent mechanism 930 is placed above at the removal position by the control of operation control circuit 940 resulting from the initial state.

When the chemical solution container 400 is inserted into the opening 929 of container holding mechanism 920 from above, the chemical solution container 400 is put on the container ascent/descent mechanism 930 and thus held at the removal position. Since the container holding mechanism 920 is locked at the retraction position by the placement lock mechanism 923, the chemical solution container 400 is not put, while the container holding mechanism 920 is displaced from the retraction position.

When the container holding mechanism 920 is appropriately placed at the retraction position, the placement is sensed by the retraction position sensor 312, to allow the container ascent/descent mechanism 930 to be operable. Then, the operator makes entry to lower the container to the operation panel 941, and the chemical solution container 400 is lowered to the holding position from the removal position together with container ascent/descent mechanism 930. As shown in Fig. 14(b), the chemical solution container 400 is held by the container holding mechanism 920, at the retraction position.

The chemical solution container 400 weighs much since container body 401 is surrounded by shield cover 411 and shield cap 412 made of thick lead to block radiation as described above. In the tube connection apparatus 900, the chemical solution container 400 is lowered smoothly to the holding position of container holding mechanism 920 by the container ascent/descent mechanism 930 as described above, it is not necessary to the lower chemical solution container 400 to collide with container holding mechanism 920 for holding.

The Container holding mechanism 920 is locked at the retraction position by the placement lock mechanism 923, in the initial state as described above, and the container ascent/descent mechanism 930 is not operated if it is displaced from the retraction position. Thus, the chemical solution container 400 is not put or lowered on the container ascent/descent mechanism 930, while the container holding mechanism 920 is displaced from the retraction position, thereby appropriately holding chemical solution container 400 by container holding mechanism 920.

After the completion of the holding, for example, the operator manually detaches the shield cap 412 from chemical solution container 400, and pinches the moving guide knob 921 of container holding mechanism 920 together with lock release knob 925 with his fingers. Then, the holding lowers lock release knob 925 to release the lock of container holding mechanism 920 with placement lock mechanism 923, so that the container holding mechanism 920 is movable.

Then, the operator manually retracts the moving guide knob 921 to slide the container holding mechanism 920 to the insertion position from the retraction position as shown in Fig. 14(c). Since the retraction position sensor 312 fails to container holding mechanism 920 at this point, the container ascent/descent mechanism 930 is fixed at the holding position and is inoperable.

When the container holding mechanism 920 is moved to the insertion position, the container holding mechanism 920 is locked by the placement lock mechanism 924 and is sensed by insertion position sensor 313. The holder moving mechanism 320 is placed above at the retraction position, before the container holding mechanism 920 is placed at the insertion position and is sensed by insertion position sensor 313. When the container holding mechanism 920 is placed at the insertion position and is sensed by insertion position sensor 313, the holder moving mechanism 320 is operable.

The operator makes entry to start insertion by operating the panel 941. Then, as shown in Fig. 14(d), the holder moving mechanism 320 lowers the short/long needle members 341, 342 to the insertion position from the retraction position and connects them to chemical solution container 400. Since the short/long needle members 341, 342 are inserted, while the container holding mechanism 920 is locked at the insertion position by placement lock mechanism 924 and is sensed by insertion position sensor 313, short/long needle members 341, 342 are not inserted, while the chemical solution container 400 is displaced from the insertion position.

After the short/long needle members 341, 342 are inserted into chemical solution container 400 by holder moving mechanism 320 as described above, this is transmitted from tube connection apparatus 900 to chemical solution injector 100 which then performs the chemical solution injection. Upon completion of the chemical solution injection, for example, the operator makes entry to release the insertion to operation panel 941. As shown in Fig. 14(c), short/long needle members 341, 342 are raised to the retraction position form the insertion position by holder moving mechanism 320 and separated from chemical solution container 400.

In this state, when the operator pinches the moving guide knob 921 of container holding mechanism 920 together with lock release knob 925, the lock with placement lock mechanism 924 is released, to allow the container holding mechanism 920 to be movable. The operator manually moves forward the moving guide knob 921. Then, as shown in Fig. 14(d), container holding mechanism 920 is slid to the retraction position from the insertion position.

Container holding mechanism 920 moved to the retraction position is locked by placement lock mechanism 923 and is sensed by retraction position sensor 312. In response to the sensing, container ascent/descent mechanism 930 is operable. When the operator makes entry to raise the container to operation panel 941, for example, chemical solution container 400 at the retraction position is raised by container ascent/descent mechanism 930 as shown in Fig. 14(a). In this manner, chemical solution container 400 is raised to the removal position from the holding position of container holding mechanism 920, and heavy chemical solution container 400 can be taken out easily.

In tube connection apparatus 900 described above, chemical solution container 400 is vertically moved at the retraction position by container ascent/descent mechanism 930 as described above, so that chemical solution container 400 can be readily detached and mounted. Since container holding mechanism 920 is locked at the retraction position and the insertion position by placement lock mechanisms 923, 924, chemical solution container 400 is not put while container holding mechanism 920 is displaced from the retraction position, and short/long needle members 341, 342 are not inserted while chemical solution container 400 is displaced from the insertion position.

Placement lock mechanisms 923, 924 described above are released when lock release knob 925 is pinched, and lock release knob 925 is pinched together with moving guide knob 921, which is used for manually moving container holding mechanism 920. It is thus possible to easily perform the operation of releasing the lock and moving container holding mechanism 920.

In addition, the container ascent/descent mechanism 930 is operable only when the container holding mechanism 920 is positioned at the retraction position, so that the container ascent/descent mechanism 930 is not operable in an inappropriate state. Since the container ascent/descent mechanism 930 is positioned at the holding position when the container holding mechanism 920 is not placed at the retraction position, the chemical solution container 400,which is moved forward and rearward together with the container holding mechanism 920 does not collide with container ascent/descent mechanism 930.

Similarly, since the holder moving mechanism 320 is operative only when the container holding mechanism 920 is positioned at the insertion position, so that it is not operable in an inappropriate state. Since the holder moving mechanism 320 is positioned at the retraction position, when the container holding mechanism 920 is not placed at the insertion position, the chemical solution container 400, which is moved forward and rearward together with container holding mechanism 920 does not collide with short/long needle members 341, 342.

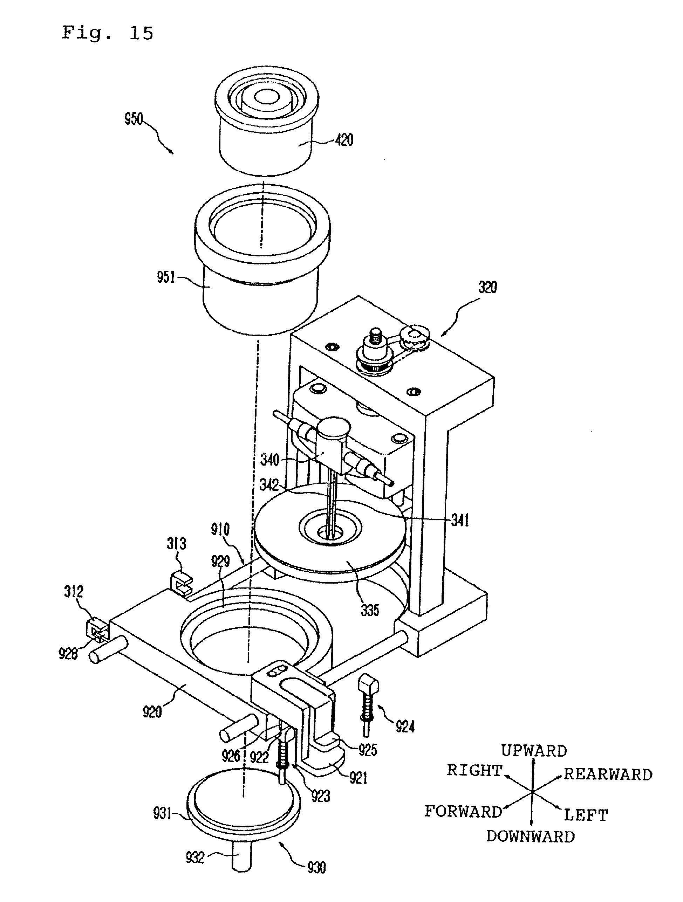

In the above embodiment, chemical solution container 400 of the given size is mounted directly on container holding mechanism 315 of tube connection apparatus 300. For example, as in tube connection apparatus 950 illustrated in Fig. 15, chemical solution container 400 of the given size may be put directly on container holding mechanism 920 and chemical solution container 420 of a size smaller than the given size may be put on container holding mechanism 920 by using dedicated container adapter 951.

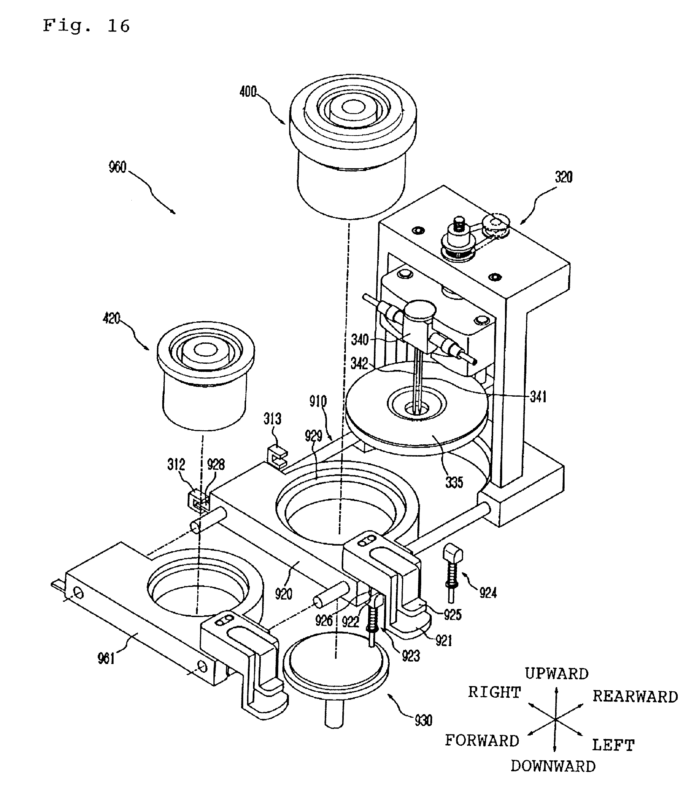

As in tube connection apparatus 960 illustrated in Fig. 16, container holding mechanisms 920, 961 may be formed for chemical solution container 400, 420 of various sizes such that container holding mechanisms 920, 961 are removably put on container moving mechanism 910.

## Claims

1. A tube connection apparatus for connecting a tube member to a chemical solution container which contains a chemical solution, and an opening of a container body of the chemical solution container being sealed by an elastic member, comprising:
a container holding mechanism for holding the chemical solution container removably, with the opening located above;
a short tubular needle member having a proximal end and a distal end, the proximal end being connected removably to a liquid supply tube in which a liquid with a specific gravity lower than that of the chemical solution flows, the distal end penetrates the elastic member;
a long tubular needle member having a proximal end and a distal end, the proximal end being connected removably to a chemical solution injection tube in which the chemical solution flows, the distal end penetrates the elastic member;
a holder member for holding the short needle member and the long needle member, with their proximal ends located above and their distal ends located below;
a holder moving mechanism for moving the short needle member and the long needle member vertically, together with the holder member, between a retraction position above and an insertion position below; and
a container moving mechanism for supporting the chemical solution container together with the container holding mechanism to be movable horizontally, between an insertion position where the chemical solution container faces the short needle member and the long needle member from below at a insertion position ,and, a retraction position where the chemical solution container does not face them.

2. The tube connection apparatus according to claim 1, wherein the container moving mechanism uses driving force of a driving source to horizontally move the chemical solution container together with the container holding mechanism.

3. The tube connection apparatus according to claim 1, whereof the container moving mechanism includes:
a placement lock mechanism for locking the chemical solution container together with the container holding mechanism, at the retraction position and the insertion position,
a moving guide knob formed integrally with the container holding mechanism and pinched by fingers for use,
a lock release knob supported on the container holding mechanism movably, and pinched by the fingers together with the moving guide knob, and
a lock release mechanism for releasing the lock of the placement lock mechanism, when the lock release knob is pinched together with the moving guide knob.

4. The tube connection apparatus according to claim 2, further comprising an operation control means for controlling the operation of the container moving mechanism and the holder moving mechanism to sequentially produce following states, in a forward and rearward order:
a retraction state in which the chemical solution container, the short needle member and the long needle members are placed at the retraction positions,
a standby state in which the chemical solution container is located at the insertion position and the short needle member and the long needle members are located at the retraction positions, and
an insertion state in which the chemical solution container, the short needle member and the long needle members are located at the insertion positions.

5. The tube connection apparatus according to any one of claims 1 to 3, further comprising:
a position sensing means for sensing whether the chemical solution container is located at the retraction position or the insertion position; and
a operation control means for controlling the operation of the holder moving mechanism, based on the result of the position sensing means, to hold the long needle member and the short needle member at the retraction position, when the chemical solution container is located at the retraction position, and allow the long needle member and the short needle member to move between the retraction position and the insertion position, only when the chemical solution container is placed at the insertion position.

6. The tube connection apparatus according to any one of claims 1 to 5, further comprising a container ascent/descent mechanism, which support the chemical solution container to be movable vertically, between a holding position where the chemical solution container is held by the container holding mechanism at the retraction position and a removal position above the holding position.

7. The tube connection apparatus according to claim 2, further comprising:
a container ascent/descent mechanism, which support the chemical solution container to be movable vertically between a holding position where the chemical solution container is held by the container holding mechanism at the retraction position and a removal position above the holding position,
a operation control means for controlling the operation of the container ascent/descent mechanism, the container moving mechanism, and the holder moving mechanism to sequentially produce following statuses, in a forward and rearward order,
a removal state in which the chemical solution container is located at the removal position at the retraction position, and the short needle member and the long needle member are located at the retraction position,
a retraction state in which the chemical solution container is located at the holding position at the retraction position, and the short needle member and the long needle members are placed at the retraction positions,
a standby state in which the chemical solution container is located at the insertion position, and the short needle member and the long needle members are located at the retraction positions, and
an insertion state in which chemical solution container, the short needle member, and the long needle members are located at the insertion positions.

8. The tube connection apparatus according to any one of claims 1 to 3, further comprising:
a container ascent/descent mechanism, which support the chemical solution container to be movable vertically, between a holding position where the chemical solution container is held by the container holding mechanism at the retraction and a removable position above the holding position,
a position sensing means for sensing whether the chemical solution container is located at the retraction position or the insertion position; and
an operation control means for controlling the operation of the container ascent/descent mechanism and the holder moving mechanism ,based on the result of the position sensing means, to allow the container ascent/descent mechanism to be operable and place the long needle member and the short needle member at the retraction position ,when the chemical solution container is located at the retraction position ,and allow the container ascent/descent mechanism to be at the holding position and allow the long needle member and the short needle member to move to the retraction position and the insertion position, when the chemical solution container is placed at the insertion position.

9. The tube connection apparatus according to any one of claims 1 to 8, wherein the chemical solution container contains a radioactive contrast medium as the chemical solution,
the tube connection apparatus further comprising a shield member for shielding the radiation of the contrast medium, at a position opposite to an opening in the chemical solution container placed at the insertion position.

10. The tube connection apparatus according to any one of claims 1 to 8, wherein the chemical solution container contains a radioactive contrast medium as the chemical solution, the tube connection apparatus further comprising a shield member for shielding the radiation of the contrast medium by closing an opening in the chemical solution container placed at the insertion position, the shield member being formed integrally with the holder member.

11. The tube connection apparatus according to any one of claims 1 to 8, wherein the chemical solution container contains a radioactive contrast medium as the chemical solution, the tube connection apparatus further comprising:
a shield member for shielding the radiation of the contrast medium by closing an opening in the chemical solution container, the shield member having a opening through which the short needle member and the long needle member are inserted; and
a shield moving mechanism for vertically moving the shield member to a retraction position where the shield member is spaced from and opposite to the chemical solution container at the insertion position from above and to an insertion position where the shield member abuts on the chemical solution container.

12. The tube connection apparatus according to claim 4 or 7, wherein the chemical solution container contains a radioactive contrast medium as the chemical solution, the tube connection apparatus further comprising:
a shield member for shielding the radiation of the contrast medium by closing an opening in the chemical solution container, the shield member having an opening through which the short needle member and the long needle member are inserted; and
a shield moving mechanism for vertically moving the shield member to a retraction position where the shield member is spaced from and opposite to the chemical solution container at the insertion position from above and to an insertion position where the shield member abuts on the chemical solution container, wherein the operation control means controls the operation of the shield moving mechanism to produce a shield state where the chemical solution container and the shield member are placed at the insertion position and the short needle member and the long needle member are located at the retraction position in the transition between the standby state and the insertion state.

13. The tube connection apparatus according to claim 5 or 8, wherein the chemical solution container contains a radioactive contrast medium as the chemical solution, the tube connection apparatus further comprising:
a shield member for shielding the radiation of the contrast medium by closing an opening in the chemical solution container, the shield member having an opening through which the short needle member and the long needle member are inserted; and
a shield moving mechanism for vertically moving the shield member to a retraction position where the shield member is spaced from and opposite to the chemical solution container at the insertion position from above and to an insertion position where the shield member abuts on the chemical solution container wherein the operation control means controls the operation of the shield moving mechanism to place the shield member at the retraction position when the chemical solution container is placed at the retraction position and to allow the shield member to be movable to the retraction position and to the insertion position when the chemical solution container is placed at the insertion position.

14. The tube connection apparatus according to claim 9, wherein the chemical solution container includes a container body containing the contrast medium, a shield cover having an opened upper surface through which the container body is inserted into the chemical solution container to shield the radiation of the contrast medium, and a shield cap removably put on the opening of the shield cover to shield the radiation,
the container holding mechanism holds the container body together with the shield cover, and
the shield member is opposite to the opening of the shield cover from which the shied cap is detached.

15. The tube connection apparatus according to any one of claims 10 to 13, wherein the chemical solution container includes a container body containing the contrast medium, a shield cover having an opened upper surface through which the container body is inserted into the chemical solution container to shield the radiation of the contrast medium, and a shield cap removably put on the opening of the shield cover to shield the radiation,
the container holding mechanism holds the container body together with the shield cover, and
the shield member closes the opening of the shield cover from which the shied cap is detached.

16. The tube connection apparatus according to claim 9, wherein the chemical solution container is removably placed in the container holding mechanism from an opening in an upper surface of the container holding mechanism, to block the radiation of the contrast medium, and the shield member is opposite to the opening of the container holding mechanism.

17. The tube connection apparatus according to any one of claims 10 to 13, wherein the chemical solution container is removably placed in the container holding mechanism from an opening in an upper surface of the container holding mechanism, to block the radiation of the contrast medium, and the shield member closes the opening of the container holding mechanism.

18. The tube connection apparatus according to any one of claims 1 to 17, wherein the short needle member and the long needle member are removably put together with the holder member.

19. The tube connection apparatus according to any one of claims 1 to 18, wherein the short needle member and the long needle member have an insertion imaging means for imaging a position where the members are inserted into the elastic member.

20. The tube connection apparatus according to any one of claims 1 to 19, wherein the chemical solution containers of various sizes are included, and
the container holding mechanism directly holds the chemical solution container of the maximum size,
the tube connection apparatus further comprising a container adapter for allowing the container holding mechanism to hold the chemical solution container of a size other than the maximum size via the adapter.

21. The tube connection apparatus according to any one of claims 1 to 19, wherein the chemical solution containers of various sizes are included, and
a plurality of the container holding mechanisms for individually holding the chemical solution containers of the various sizes are removably supported on the container moving mechanism.

22. A chemical solution injector for injecting a chemical solution contained in a chemical solution container into a patient, an opening of a container body of the chemical solution container being sealed by an elastic member, comprising:
the tube connection apparatus according to any one of claims 1 to 21, a liquid supply tube,
a chemical solution injection tube,
a liquid container containing a liquid and connected to a proximal end of the liquid supply tube, and
a liquid pressing mechanism for pressing the liquid in the liquid container to the chemical solution container via the liquid supply tube.

23. A chemical solution injector for injecting a chemical solution contained in a chemical solution container into a patient, an opening of a container body of the chemical solution container being sealed by an elastic member, comprising:
the tube connection apparatus according to any one of claims 4, 7, and 12;
a liquid supply tube;
a chemical solution injection tube;
a liquid container containing the liquid and connected to a proximal end of the liquid supply tube;
a liquid pressing mechanism for pressing the liquid in the liquid container to the chemical solution container via the liquid supply tube; and
a pressing control means for allowing the liquid pressing mechanism to be operative only when the tube connection apparatus is in the insertion state.

24. A chemical solution injector for injecting a chemical solution contained in a chemical solution container into a patient, an opening of a container body of the chemical solution container being sealed by an elastic member, comprising:
the tube connection apparatus according to any one of claims 5, 8, and 13;
a liquid supply tube;
a chemical solution injection tube;
a liquid container containing the liquid and connected to a proximal end of the liquid supply tube;
a liquid pressing mechanism for pressing the liquid in the liquid container to the chemical solution container via the liquid supply tube; and
a pressing control means for allowing the liquid pressing mechanism to be operative only when the tube connection apparatus places the short needle member and the long needle member at the insertion position.

25. A chemical solution injector for injecting a chemical solution contained in a chemical solution container into a patient, an opening in a container body of the chemical solution container being sealed by an elastic member, comprising:
the tube connection apparatus according to claim 19;
a liquid supply tube;
a chemical solution injection tube;
a liquid container containing the liquid and connected to a proximal end of the liquid supply tube;
a liquid pressing mechanism for pressing the liquid in the liquid container to the chemical solution container via the liquid supply tube;
an image display means for outputting and displaying various types of data including an operation state of the liquid pressing mechanism; and
a display control means for allowing the image display means to output and display an image taken by the insertion imaging means.

26. A chemical solution injector for injecting a chemical solution contained in a chemical solution container into a patient, an opening of a container body of the chemical solution container being sealed by an elastic member, comprising:
a liquid supply tube in which a given liquid with a specific gravity lower than that of the chemical solution flows;
a chemical solution injection tube in which the chemical solution flows, and being removably connected to the patient;
a liquid container containing the liquid and connected to a proximal end of the liquid supply tube;
a liquid pressing mechanism for pressing the liquid in the liquid container to the chemical solution container via the liquid supply tube;
a data communicating means for communicating data with the connection apparatus according to any one of claims 4, 7, and 12; and
a pressing control means for allowing the liquid pressing mechanism to be operative only when the tube connection apparatus is in the insertion state.

27. A chemical solution injector for injecting a chemical solution contained in a chemical solution container into a patient, an opening of a container body of the chemical solution container being sealed by an elastic member, comprising:
a liquid supply tube in which a given liquid with a specific gravity lower than that of the chemical solution flows;
a chemical solution injection tube in which the chemical solution flows, and being removably connected to the patient;
a liquid container containing the liquid and connected to a proximal end of the liquid supply tube;
a liquid pressing mechanism for pressing the liquid in the liquid container to the chemical solution container via the liquid supply tube;
a data communicating means for communicating data with the connection apparatus according to any one of claims 5, 8, and 13; and
a pressing control means for allowing the liquid pressing mechanism to be operative only when the short needle member and the long needle member are positioned at the insertion position.

28. A chemical solution injector for injecting a chemical solution contained in a chemical solution container into a patient, an opening of a container body of the chemical solution container being sealed by an elastic member, comprising:
a liquid supply tube in which a given liquid with a specific gravity lower than that of the chemical solution flows;
a chemical solution injection tube in which the chemical solution flows, and being removably connected to the patient;
a liquid container containing the liquid and connected to a proximal end of the liquid supply tube;
a liquid pressing mechanism for pressing the liquid in the liquid container to the chemical solution container via the liquid supply tube;
an image display means for outputting and displaying various types of data including an operation state of the liquid pressing mechanism;
a data communicating means for communicating data with the connection apparatus according to claim 19; and
a display control means for allowing the image display means to output and display an image taken by the insertion imaging means.

29. The chemical solution injector according to any one of claims 22 to 28, further comprising:
an air-bubble detection sensor for detecting an air-bubble mixed in the chemical solution flowing in the chemical solution injection tube; and
an injection control means for forcedly stopping the liquid pressing mechanism when the air-bubble is detected.

30. The chemical solution injector according to any one of claims 22 to 29, further comprising:
an air-bubble detection sensor for detecting an air-bubble mixed in the chemical solution flowing in the chemical solution injection tube; and
an injection block mechanism for blocking the chemical solution injection tube when the air-bubble is detected.

31. The chemical solution injector according to any one of claims 22 to 30, further comprising an air-bubble removing mechanism for removing an air-bubble from the chemical solution flowing in the chemical solution injection tube.

32. The chemical solution injector according to any one of claims 22 to 31, further comprising:
an exhaustion detecting means for detecting exhaustion of the liquid pressed into the chemical solution container from the liquid container via the liquid supply tube; and
an injection control means for forcedly stopping the liquid pressing mechanism when the exhaustion is detected.

33. The chemical solution injector according to any one of claims 22 to 32, further comprising:
an exhaustion detecting means for detecting exhaustion of the liquid pressed into the chemical solution container from the liquid container via the liquid supply tube; and
a supply block mechanism for blocking the liquid supply tube when the exhaustion is detected.

34. The chemical solution injector according to any one of claims 22 to 33, wherein the chemical solution container is formed of a chemical solution syringe including a cylinder member and a piston member slidably inserted into the cylinder member, and
the liquid pressing mechanism presses the piston member into the cylinder member of the chemical solution syringe removably mounted.

35. The chemical solution injector according to any one of claims 22 to 34, further comprising:
a switch detection sensor for detecting a switch from the chemical solution to the liquid, the chemical solution being pressed into the patient from the chemical solution container via the chemical solution injection tube; and
an injection control means for forcedly stopping the liquid pressing mechanism in response to the detection of the switch.

36. The chemical solution injector according to any one of claims 22 to 35, further comprising:
a switch detection sensor for detecting a switch from the chemical solution to the liquid, the chemical solution being pressed into the patient from the chemical solution container via the chemical solution injection tube; and
an injection control means for controlling the operation of the liquid pressing mechanism in response to the detection of the switch.

37. The chemical solution injector according to claim 36, wherein, when the switch is detected, the injection control means forcedly stops the liquid pressing mechanism after a given quantity of the liquid is pressed.

38. The chemical solution injector according to claim 37, further comprising:
a body part input means for receiving data of a body part to be imaged by an imaging diagnostic apparatus which shoots a diagnostic image of a body part of the patient injected with the chemical solution,
a quantity storing means for storing data of a quantity of the liquid for each body part to be imaged, and
a quantity reading means for reading data of the quantity when data of the body part to be imaged is input,
wherein, when the switch is detected, the injection control means forcedly stops the liquid pressing mechanism after the liquid of the read quantity is pressed.

39. The chemical solution injector according to any one of claims 35 to 38, wherein the chemical solution container contains a radioactive contrast medium as the chemical solution, and
the switch detection sensor detects a change from the chemical solution to the liquid based on the presence or absence of the radiation.

40. An imaging diagnostic system comprising:
the chemical solution injector according to any one of claims 22 to 39, and
an imaging diagnostic apparatus for shooting a diagnostic image of a body part to be imaged in the patient injected with the chemical solution,
wherein the chemical solution injector further includes a completion detecting means for detecting completion of injection of the chemical solution, and
a data transmitting means for transmitting the detected injection completion to the imaging diagnostic apparatus, and
the imaging diagnostic apparatus include,
an imaging means for shooting the diagnostic image,
a data receiving means for receiving the injection completion,
a time detecting means for detecting the lapse of a given time period when the injection completion is received, and
an imaging control means for allowing the imaging means to start shooting after the lapse of the given time period.

41. An imaging diagnostic system comprising:
the chemical solution injector according to any one of claims 22 to 39, and an imaging diagnostic apparatus for shooting a diagnostic image of a body part to be imaged in the patient injected with the chemical solution,
wherein the chemical solution injector further includes:
a completion detecting means for detecting completion of injection of the chemical solution,
a time detecting means for detecting the lapse of a given time period when the injection completion is detected, and
a data transmitting means for transmitting the detected lapse of the time to the imaging diagnostic apparatus, and
the imaging diagnostic apparatus includes,
an imaging means for shooting the diagnostic image,
a data receiving means for receiving the lapse of the time, and
an imaging control means for allowing the imaging means to start shooting when the lapse of the time is received.
